(19)

**Europäisches Patentamt**
**European Patent Office**
**Office européen des brevets**

(11) **EP 4 692 376 A1**

(12) **EUROPEAN PATENT APPLICATION**
published in accordance with Art. 153(4) EPC

(43) Date of publication:
**11.02.2026 Bulletin 2026/07**

(21) Application number: **24792722.1**

(22) Date of filing: **18.04.2024**

(51) International Patent Classification (IPC):
**C12Q 1/6888** (2018.01)    **C12M 1/34** (2006.01)

(52) Cooperative Patent Classification (CPC):
**C12M 1/34; C12Q 1/6888**

(86) International application number:
**PCT/JP2024/015385**

(87) International publication number:
**WO 2024/219450 (24.10.2024 Gazette 2024/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **18.04.2023  JP 2023068156**

(71) Applicants:
• **National University Corporation
  Hokkaido University
  Hokkaido 060-0808 (JP)**
• **Cellspect Co., Ltd.
  Morioka-shi, Iwate 020-0857 (JP)**

(72) Inventors:
• **SATOH, Hisashi
  Sapporo-shi, Hokkaido 060-0808 (JP)**
• **NAKAYA, Yuki
  Sapporo-shi, Hokkaido 060-0808 (JP)**

• **NAKAJIMA, Meri
  Sapporo-shi, Hokkaido 060-0808 (JP)**
• **HANDA, Hisazumi
  Sapporo-shi, Hokkaido 060-0808 (JP)**
• **SANARI, Kota
  Sapporo-shi, Hokkaido 060-0808 (JP)**
• **HIRANO, Reiko
  Morioka-city, Iwate 0200857 (JP)**
• **DOI, Kazuhiko
  Morioka-city, Iwate 0200857 (JP)**
• **HOJO, Wataru
  Morioka-city, Iwate 0200857 (JP)**
• **IWABUCHI, Takuya
  Morioka-city, Iwate 0200857 (JP)**
• **SAKAI, Hironori
  Morioka-city, Iwate 0200857 (JP)**

(74) Representative: **Hasegawa, Kan
  Patentanwaltskanzlei Hasegawa
  Untere Hauptstraße 56
  85354 Freising (DE)**

(54) **MICROBIAL NUCLEIC ACID DETECTION METHOD, REAGENT COMPOSITION, REAGENT KIT, MEASUREMENT SYSTEM, AND PROGRAM**

(57)    A microbial nucleic acid detection method, and the like, are provided that can measure the concentration of a target nucleic acid easily, quickly, and with high accuracy, and secure a sufficient measurement range (dynamic range). The microbial nucleic acid detection method comprises the steps of: preparing a sample mixture by: mixing a probe solution, being a gold nanoparticle probe solution in which DNA that binds specifically to a target nucleic acid is modified with gold nanoparticles, with an extract obtained by applying a nucleic acid extraction treatment to a sample; and adding at least sodium chloride and applying heat thereto; acquiring an absorption spectrum of the probe solution; acquiring an absorption spectrum of a blank solution, being a sample-free solution obtained by adding sodium chloride to the probe solution and applying heat thereto; acquiring an absorption spectrum of the sample mixture; and performing calculations to estimate the concentration of the target nucleic acid in the sample based on the absorption spectra of the probe solution, the blank solution, and the sample mixture.

EP 4 692 376 A1

# FIG.1

```
        ┌─────────────┐
        │    START    │
        └─────────────┘
               │
               ▼
   ┌──────────────────────────┐
   │ NUCLEIC ACID EXTRACTION  │─── S10
   │       TREATMENT          │
   └──────────────────────────┘
               │
               ▼
   ┌──────────────────────────┐
   │ ADD DNA PROBE SOLUTION   │
   │ TO EXTRACT, FURTHER ADD  │─── S20
   │ NaCl AND APPLY HEAT      │
   └──────────────────────────┘
               │
               ▼
   ┌──────────────────────────┐
   │ ACQUIRE ABSORPTION       │─── S30
   │ SPECTRUM                 │
   └──────────────────────────┘
               │
               ▼
   ┌──────────────────────────┐
   │ ESTIMATE NUCLEIC ACID    │─── S40
   │ CONCENTRATION            │
   └──────────────────────────┘
               │
               ▼
        ┌─────────────┐
        │     END     │
        └─────────────┘
```

## EP 4 692 376 A1

**Description**

TECHNICAL FIELD

The present invention relates to microbial nucleic acid detection methods, reagent compositions, reagent kits, measurement systems, and programs.

BACKGROUND ART

The World Health Organization (WHO) has reported that about a quarter of the people in 138 countries worldwide, or about 2 billion people, had no access to safely managed drinking water supply facilities in 2020 (cf. "Our lifetime opportunity to enable water, sanitation and hygiene for all": https://www.who.int/news/item/22-03-2023-our-lifetime-opportunity-to-enable-water-sanitation-and-hygiene-for-all). Of these, about 1.2 billion people used only basic, unsafe water supply facilities, about 282 million people had limited supplies, about 367 million people used unimproved water sources, and about 122 million people used surface water.

With regard to sanitation facilities such as toilets, only 54% of the world's population has access to safely managed facilities (i.e., facilities that can safely manage waste inside or outside the facility), and 3.6 billion people only have access to inadequate facilities. It is estimated that, in order for these people to have access to safely managed water supply and basic sanitation facilities by 2030, the rate of progression of the deployment of these facilities will need to be quadrupled. In least developed countries and vulnerable regions, the situation is even more severe, and it is said that the current rate of progression needs to be increased to 10 and 23 times, respectively. In 2020, about 44% of household wastewater was discharged into the environment without being treated safely, and inadequate sanitation facilities and wastewater treatment are sources of pathogen discharge into the environment.

The WHO estimates that, in 2016, there were 870,000 related deaths worldwide due to unsafe drinking water facilities and lack of sanitation facilities. Related deaths in the African region accounted for 45.9 per 100,000 people among its population, and this figure corresponds to a quadrupling of the world average (11.7 per 100,000 people). The European region is the least affected by such related deaths (0.3 per 100,000 people). The mortality rate in the African region is about 150 times higher than the mortality rate in the Europe region, and there is a significant imbalance between the regions.

In general, the greatest microbiological risk of drinking water relates to the ingestion of water contaminated by human or animal (including bird) feces. These feces are likely to contain bacteria, viruses, protozoa, and helminths that have pathogenicity. The main symptoms of water-borne infections are abdominal pain, diarrhea, vomiting, fever, and headache. Diarrhea causes dehydration and can therefore lead to death in poorly nourished developing countries. The WHO has formulated the Guidelines for Drinking-Water Quality, in which the microorganisms listed in Table 1 are designated as pathogenic microorganisms (cf., "WHO Guidelines for drinking-water quality: Fourth edition (Japanese version)", National Institute of Public Health, https://www.mhlw.go.jp/file/05-Shingikai-11121000-Iyakushokuhinkyoku-Soumuka/0000195447.pdf).

[Table 1]

| Bacteria | Acinetobacter | Opportunistic pathogenic bacteria that cause urinary duct infection, pneumonia, bacillemia, secondary meningitis, and wound infection |
| --- | --- | --- |
| | Aeromonas | Sepsis, in particular, wound infection and respiratory infection in immunodeficient patients |
| | Campylobacter | Relatively highly infectious even at low concentrations. Abdominal pains, diarrhea, vomiting, chills, fever |
| | Enteropathogenic Escherichia Coli | Urinary duct infection, bacillemia, meningitis, hemorrhagic colitis |
| | Legionella | Legionnaires' disease (Legionella pneumonia and Pontiac fever) |
| Protozoa | Cryptosporidium | Diarrhea, nausea, sick feeling, fever |
| | Giardia Intestinalis | Diarrhea, malabsorption |

Overall downsizing and cost reduction of water purification systems and efficient use of water resources are expected to enable more people to access safe water, especially in developing countries and localities. For this purpose, a system is desired that can quickly monitor water quality on the spot (on-site), even under various regions and environments. This is because by being able to streamline the water purification process, water can be reused even in simple membrane filtration systems, reverse osmosis membrane filtration systems, and systems similar thereto, thereby promoting the sustainable use of water resources.

Culture, PCR, latex agglutination, immunoassay, and PCR are known techniques for microbial monitoring in environmental water. In addition, for example, PTL 1 discloses an analysis method using the PCR method that simultaneously performs the step of separating nucleic acids of the pathogen in tissue fragments and the step of preparing the PCR buffer solution. In addition, PTL 2 discloses a nucleic acid primer set for distinguishing and detecting Chlamydia bacteria and a method for detecting Chlamydia bacteria using the primer set.

PRIOR ART DOCUMENTS

PATENT LITERATURE

PTL 1: JP2021-122186
PTL 2: JP2021-158989

NON-PATENT LITERATURE

NPL 1: Meri Nakajima, et al., "Development of a Paper-based Analytical Chip for the Detection of Bacterial 16S rRNA in Wastewater Samples", BUNSEKI KAGAKU, Vol. 69, No. 12, pp. 715-722 (2020)
NPL 2: Meri Nakajima et al., "Simple Assay for Colorimetric Quantification of Unamplified Bacterial 16S rRNA in Activated Sludge using Gold Nanoprobes", Chemosphere, Vol. 263, January 2021, 128331
NPL 3: Hisashi Satoh et al., "Highly sensitive and homogeneous detection of unamplified RNA based on the light scattering properties of gold nanoparticle probes", Biosensors and Bioelectronics: X, Vol. 12, December 2022, 100249

SUMMARY OF INVENTION

PROBLEMS TO BE SOLVED BY INVENTION

The culture method is a typical measurement method, but it takes a long time (e.g., 24 hours) to obtain results. There is also a problem that it cannot detect unculturable microorganisms, and/or viable but non-culturable microorganisms (VBNC bacteria). In addition, as mentioned above, there are a wide variety of pathogenic microorganisms that cause water-borne infections, so selective culture media is required for each pathogenic microorganism, which is impractical.

Alternative testing techniques to the culture method include the PCR or quantitative (real-time) PCR method. The PCR method is a technique for detecting and quantifying nucleic acids by amplifying them through polymerase chain reaction (PCR) using enzymes (DNA polymerase), and has high sensitivity and specificity. However, the PCR method is associated with problems such as the fact that the process is cumbersome so that a high level of operational capability is required, a long analysis time (e.g., 3 hours) is required, expensive consumables such as enzymes and expensive analytical instruments are required, and the like. The aforementioned PTLs 1 and 2 have similar problems because they use the PCR method.

In addition, the immunoassay and latex agglutination methods using antibodies are simple but inferior in terms of sensitivity.

Therefore, it is extremely difficult to frequently analyze the biological risks of drinking water using these conventional techniques, especially in developing countries and disaster areas. For this reason, a system that is simpler and has the equivalent detection capability as a PCR method is desired.

The inventors of the present application have focused on gold nanoparticles, designed DNA that specifically binds to the 16S rRNA gene of bacteria, and prepared gold nanoparticle probes by modifying the DNA with gold nanoparticles. Using these probes, the inventors have been developing simple, rapid, and highly accurate nucleic acid analysis methods and reagent compositions (see NPLs 1 to 3).

However, in the techniques employed by the inventors of the present application, although the concentration of the nucleic acid to be detected (target nucleic acid) can be measured easily, quickly, and even with high accuracy, there remains a problem in that the measurement range (dynamic range) of the concentration is insufficient.

The present invention has been made in view of the above, and an object thereof is to provide a microbial nucleic acid detection method, a reagent composition, a reagent kit, a measurement system, and a program that can easily, quickly, and accurately measure the concentration of a target nucleic acid and ensure a sufficient measurement range (dynamic range).

MEANS FOR SOLVING THE PROBLEMS

In order to solve the above problems, the microbial nucleic acid detection method, which is one aspect of the present invention, comprises the steps of: preparing a sample mixture by: mixing a probe solution, being a gold nanoparticle probe solution in which DNA that binds specifically to a target nucleic acid is modified with gold nanoparticles, with an extract obtained by applying a nucleic acid extraction treatment to a sample; and adding at least sodium chloride and applying heat thereto; acquiring an absorption spectrum of the probe solution; acquiring an absorption spectrum of a blank solution, being a sample-free solution obtained by adding sodium chloride to the probe solution and applying heat thereto; acquiring an absorption spectrum of the sample mixture; and performing calculations to estimate the concentration of the target nucleic acid in the sample based on the absorption spectra of the probe solution, the blank solution and the sample mixture.

In the above microbial nucleic acid detection method, in the calculation step, the concentration of the target nucleic acid in the sample may be estimated by performing curve fitting based on the absorption spectra of the probe solution, the blank solution, and the sample mixture.

In the above microbial nucleic acid detection method, in the calculation step, a least squares method may be performed so that a linear combination of a first term representing the absorption spectrum of the probe solution, a second term representing the absorption spectrum of the blank solution, and a third term representing the absorption spectrum of the sample mixture is closest to the third term representing the absorption spectrum of the sample mixture, and the concentration of the target nucleic acid may be estimated based on a coefficient of the third term calculated by the least squares method.

In the above microbial nucleic acid detection method, the preparation step may include further adding bovine serum albumin.

In the above microbial nucleic acid detection method, the concentration of the bovine serum albumin may be greater than 0.01 mg/L and less than 1 mg/L in the sample mixture.

The reagent composition, which is another aspect of the present invention, is a reagent composition to be used for detecting a microorganism in a sample, comprising: a probe solution, being a gold nanoparticle probe solution in which DNA that binds specifically to a target nucleic acid is modified with gold nanoparticles; and a sodium chloride solution, wherein the reagent composition is used to prepare: a sample mixture obtained by mixing the probe solution with an extract obtained by applying a nucleic acid extraction treatment to the sample, and adding at least sodium chloride and applying heat thereto; and a blank solution, being a sample-free solution obtained by adding sodium chloride to the probe solution and applying heat thereto, and the reagent composition is for estimating the concentration of the target nucleic acid in the sample by calculations based on absorption spectra of the sample mixture, the blank solution, and the probe solution.

The above reagent composition may further comprise bovine serum albumin.

The reagent kit, which is another aspect of the present invention, is a reagent kit to be used for detecting a microorganism in a sample, comprising: a probe solution, being a gold nanoparticle probe solution in which DNA that binds specifically to a target nucleic acid is modified with gold nanoparticles; and a sodium chloride solution, wherein each of the probe solution and the sodium solution is enclosed in a predetermined amount, and the reagent kit is used to prepare: a sample mixture obtained by mixing the probe solution with an extract obtained by applying a nucleic acid extraction treatment to the sample, and adding at least sodium chloride and applying heat thereto; and a blank solution, being a sample-free solution obtained by adding sodium chloride to the probe solution and applying heat thereto, and the reagent kit is for estimating the concentration of the target nucleic acid in the sample by calculations based on absorption spectra of the sample mixture, the blank solution, and the probe solution.

The above reagent kit may further comprise bovine serum albumin enclosed in a predetermined amount.

The measurement system, which is another aspect of the present invention, comprises: a measurement unit that acquires an absorption spectrum of a sample mixture, the sample mixture being obtained by mixing a probe solution, being a gold nanoparticle probe solution in which DNA that binds specifically to a target nucleic acid is modified with gold nanoparticles, with an extract obtained by applying a nucleic acid extraction treatment to a sample, and adding at least sodium chloride and applying heat thereto; and a calculation unit that estimates the concentration of the target nucleic acid in the sample based on an absorption spectrum of the probe solution, an absorption spectrum of a blank solution, being a sample-free solution obtained by adding sodium chloride to the probe solution and applying heat thereto, and the absorption spectrum of the sample mixture.

In the above measurement system, the measurement unit may further measure an absorption spectrum of the probe solution and an absorption spectrum of the blank solution, and the calculation unit may estimate the concentration of the target nucleic acid in the sample based on the absorption spectrum of the probe solution, the absorption spectrum of the blank solution, and the absorption spectrum of the sample mixture, all of which are measured by the measurement unit.

The above measurement system may further comprise a memory unit that stores at least one of the absorption spectrum of the probe solution or the absorption spectrum of the blank solution, and the calculation unit may read at least one of the absorption spectrum of the probe solution or the absorption spectrum of the blank solution from the memory unit, and estimate the concentration of the target nucleic acid in the sample using the read absorption spectrum.

In the above measurement system, the calculation unit may estimate the concentration of the target nucleic acid the sample by performing curve fitting based on the absorption spectra of the probe solution, the blank solution, and the sample mixture.

The program, which is another aspect of the present invention, is for causing a computer to perform: a step of acquiring an absorption spectrum of a sample mixture, the sample mixture being obtained by mixing a probe solution, being a gold nanoparticle probe solution in which DNA that binds specifically to a target nucleic acid is modified with gold nanoparticles, with an extract obtained by applying a nucleic acid extraction treatment to a sample, and adding at least sodium chloride and applying heat thereto; and a step of estimating the concentration of the target nucleic acid in the sample based on an absorption spectrum of the probe solution, an absorption spectrum of a blank solution, being a sample-free solution obtained by adding sodium chloride to the probe solution and applying heat thereto, and the absorption step of the sample mixture.

EFFECT OF INVENTION

According to the invention, the concentration of a target nucleic acid to be detected can be measured easily, quickly, and with high accuracy, and a sufficient measurement range (dynamic range) can be secured.

BRIEF DESCRIPTION OF DRAWINGS

FIG. 1 is a flowchart illustrating a basic nucleic acid detection method in an embodiment of the present invention.
FIG. 2 is a schematic diagram for describing the principle of nucleic acid detection in an embodiment of the present invention.
FIG. 3 is a schematic diagram for describing the principle of nucleic acid detection in an embodiment of the present invention.
FIG. 4 is a schematic diagram for describing a nucleic acid detection method in a first embodiment of the present invention.
FIG. 5 is a graph showing the results of Example 1.
FIG. 6 is a schematic diagram for describing a nucleic acid detection method in a second embodiment of the present invention.
FIG. 7 is a graph showing a comparative example of Example 2.
FIG. 8 is a graph showing the experimental results of Example 2.
FIG. 9 is a block diagram illustrating a schematic configuration of a measurement system according to a third embodiment of the present invention.

EMBODIMENTS OF INVENTION

Hereinafter, the microbial nucleic acid detection method, reagent composition, reagent kit, measurement system, and program will be described with reference to the drawings. It should be noted that the present invention is not limited by these embodiments. In the description of each drawing, the same parts are denoted by the same reference numbers.

The drawings referred to in the following description merely represent, in a schematic manner, the shape, size, and positional relationship, to the extent that the content of the present invention may be understood. In other words, the present invention is not limited only to the shapes, sizes, and positional relationships illustrated in the respective figures. In addition, the drawings may also include, among the same, parts having different dimensional relationships and ratios from each other.

The microbial nucleic acid detection method according to the embodiments described below is a method to quantify microorganisms in a sample, such as environmental water and sludge, by extracting nucleic acids from the sample, mixing them with a gold nanoparticle probe solution, and measuring the absorbance of this solution. According to the embodiments described below, it is possible to quantify nucleic acids easily, quickly, and with high accuracy without the need to amplify the extracted nucleic acids.

In the present application, microorganisms include: prokaryotes including bacteria and archaea; eukaryotes including protists, algae, and fungi; and viruses.

(Principle of Nucleic Acid Detection)

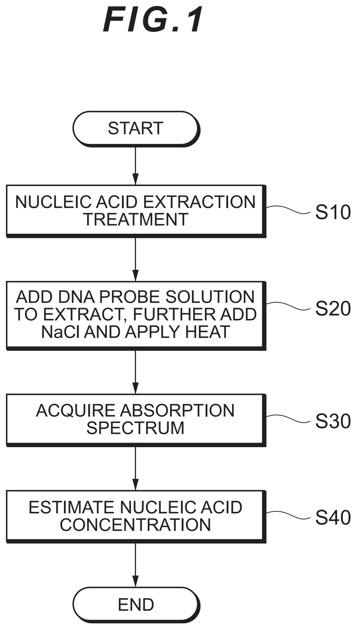

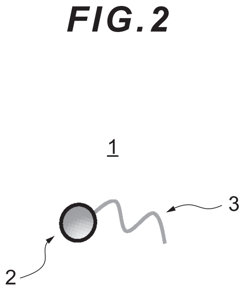

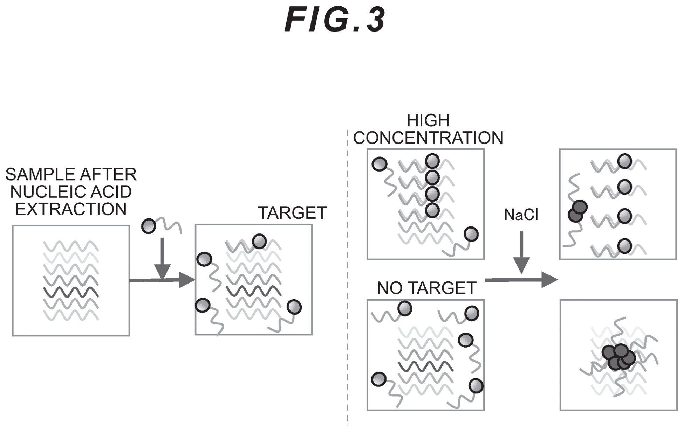

FIG. 1 is a flowchart illustrating a basic nucleic acid detection method in each embodiment of the present invention. FIGS. 2 and 3 are schematic diagrams for describing the principle of nucleic acid detection in each embodiment of the present invention. In the following embodiments, a gold nanoparticle probe (also hereinafter simply referred to as a "probe") is prepared, in which a DNA strand complementary to the base sequence of a target nucleic acid to be detected is modified with gold nanoparticles (AuNP), and the solution containing such gold nanoparticle probe is used as the reagent composition. The probe (1) illustrated in FIG. 2 is obtained by modifying a complementary DNA strand (3) that binds specifically to the target nucleic acid with a gold nanoparticle (2). The target nucleic acids (target genes) are not particularly limited, and the principle of nucleic acid detection described below can be applied using various bacterial and viral RNA genes, such as the 16S rRNA gene of bacteria, the GI or GII type RNA gene of norovirus, and the like, as targets.

First, a treatment to extract nucleic acids from the sample is performed (pretreatment step S10). It should be noted that the methods to extract nucleic acids are not particularly limited, and any publicly-known method may be applied.

Next, the gold nanoparticle probe solution (also hereinafter simply referred to as the "probe solution") is added to the nucleic acid extract (also hereinafter simply referred to as the "extract") from the sample, and sodium chloride (NaCl) is further added and heat is applied thereto (preparation step S20). The heating condition can be, for example, at 95°C for 30 seconds. If the target nucleic acid is contained in the extract, the probe would be hybridized with the target nucleic acid in the mixture.

Next, the absorption spectrum of the mixture is acquired (measurement step S30) and the nucleic acid concentration is estimated based on this absorption spectrum (calculation step S40).

Here, as shown in FIG. 3, when the probe is hybridized, the target nucleic acid covers the gold nanoparticles of the probe, inhibiting the aggregation of gold nanoparticles by sodium (Na) ions. Therefore, if the target nucleic acid concentration in the extract is high (that is, if the target (microorganism) is present in the sample at high concentration), the original red color of the gold nanoparticles is maintained in the mixture. On the other hand, if the target nucleic acid concentration in the extract is low, the gold nanoparticles tend to be aggregated by the Na ions, which causes aggregation. As a result, the original color of the gold nanoparticles is no longer observed. Therefore, the state of the gold nanoparticles, in other words, the degree of hybridization of the target nucleic acid to the probe, can be estimated by analyzing the spectrum in the mixture.

(Reagent Composition)

The reagent composition used in the embodiments described below is a reagent composition used to detect microorganisms in a sample, and comprises a probe solution and a sodium chloride solution. The probe solution is a gold nanoparticle probe solution in which DNA that binds specifically to a target nucleic acid is modified with gold nanoparticles. Such reagent composition is used to prepare a sample mixture obtained by mixing the probe solution with the extract obtained by applying a nucleic acid extraction treatment to the sample, and adding at least sodium chloride and applying heat thereto. In addition, the reagent composition may further contain bovine serum albumin.

The reagent composition may also be used to prepare a blank solution, which is a sample-free solution obtained by adding sodium chloride to the probe solution and applying heat thereto. In this case, the reagent composition is for estimating the target nucleic acid concentration in the sample by calculations based on the absorption spectra of the sample mixture, the blank solution, and the probe solution.

As mentioned above, the types of target nucleic acids are not particularly limited, and a reagent composition can be composed of a gold nanoparticle probe in which a complementary DNA strand that binds specifically to the target nucleic acid is modified with gold nanoparticles, targeting RNA genes of various bacteria and viruses. By combining such reagent composition with a general-purpose nucleic acid extractor, it is possible to realize one-stop (i.e., dispensing with cumbersome operations), simple, and PCR method-equivalent microbial nucleic acid detection.

(First Embodiment)

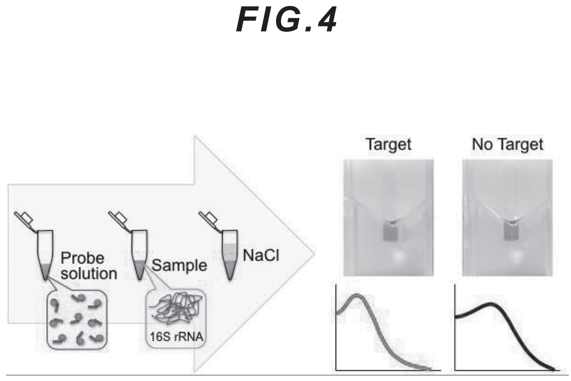

Next, a nucleic acid detection method according to a first embodiment of the present invention will be described. FIG. 4 is a schematic diagram for describing a nucleic acid detection method in a first embodiment of the present invention.

In the first embodiment, bovine serum albumin (BSA) is also added when sodium chloride is added to the mixture of the sample extract and the probe solution (see step S20). Again, in this case, the heating condition can be set at 95°C for 30 seconds. Alternatively, heat may be applied at 95°C for 30 seconds and then at 59°C for 1 minute.

The absorbance ratio can be used for the method for estimating nucleic acid concentration (step S40).

As mentioned above, here, when the probe is hybridized, the aggregation of gold nanoparticles by sodium (Na) ions is inhibited. Therefore, as shown in FIG. 4, if the target nucleic acid concentration in the extract is high, the original red color of the gold nanoparticles is maintained in the sample mixture, and the absorbance peak is observed at around a 550 nm wavelength.

On the other hand, as the target nucleic acid concentration in the extract decreases, the gold nanoparticles tend to be aggregated by Na ions, and the more aggregated, the more the increase of absorbance around a 650 nm wavelength in the sample mixture.

Thus, the absorbance ratio $R_{650}/R_{550}$, which is obtained by dividing the absorbance $A_{650}$ at the 650 nm wavelength in the sample mixture by the absorbance $A_{550}$ at the 550 nm wavelength, can be said to represent the aggregation degree of gold nanoparticles, in other words, the target nucleic acid concentration. That is, the higher the absorbance ratio, the lower the target nucleic acid concentration, and a negative correlation is observed between the two.

Therefore, the target nucleic acid concentration can be estimated by extracting absorbances at the 550 nm and 650 nm wavelengths from the spectrum acquired in step S30, calculating the ratio $R_{650}/R_{550}$ of these absorbances, and comparing the absorbance ratio with the previously acquired calibration curve, or substituting the absorbance ratio into the previously acquired estimation formula. It should be noted that, in the first embodiment, because the wavelengths used are the above two wavelengths, only these wavelengths may be acquired by, for example, using a microplate reader in step S30.

Here, in the first embodiment, BSA is further added to the sample mixture. As a result, BSA molecules cling around the probe, which tends to inhibit the aggregation of gold nanoparticles by Na ions. Therefore, even if the target nucleic acid concentration is low, the absorbance peak around the 550 nm wavelength in the mixture is higher compared to the BSA-free case. This allows for the measurement of the target nucleic acid concentration to be made even in samples with a low target nucleic acid concentration. In other words, this allows for the measurement range to be extended to include the lower concentration side.

(Example 1)

Experiments were conducted in which a gold nanoparticle probe was prepared that binds specifically to target nucleic acids, and the target nucleic acids in the samples were detected using this gold nanoparticle probe. In Example 1, 16S rRNA genes were targeted, and a gold nanoparticle probe was prepared in which a DNA strand complementary to the base sequence of the 16S rRNA genes was modified with gold nanoparticles.

1. Preparation of Probe Solution

The gold nanoparticle probe was prepared with reference to prior research (see, Liu et al., "Preparation of aptamer-linked gold nanoparticle purple aggregates for colorimetric sensing of analytes" Nat. Protoc. 1, 246e252. https://doi.org/10.1038/nprot.2006.38., X. Zhang et al., "Instantaneous and Quantitative Functionalization of Gold Nanoparticles with Thiolated DNA Using a pH-Assisted and Surfactant-Free Route" J. Am. Chem. Soc., 134, 7266 (2012) https://doi.org/10.1021/ja3014055).

First, a 100 mM acetate buffer with a pH of 5.2 was prepared. This was prepared by mixing 0.107 mL acetate and 0.677 g sodium acetate and adjusting the volume to 100 mL. Next, a 10 mM solution of tris (2-carboxyethyl) phosphine (TCEP) was prepared. This was prepared by measuring and taking out 1 to 3 mg of TCEP and dissolving it in ultrapure water (Milli-Q water). Since the TCEP solution is unstable, it was prepared each time the probe was made.

Using the aforementioned solutions, 100 mM acetate buffer and 10 mM TCEP solution were mixed in a 9:1 ratio to prepare a deprotection buffer. 3 $\mu$L of 10 $\mu$M DNA solution was added to 27 $\mu$L of the deprotection buffer and the mixture was sufficiently vortexed (stirred using a vortex mixer (the same applies hereinafter)). After spinning down, the mixture was left to stand at room temperature for one hour and the thiol group of the DNA was deprotected.

3 $\mu$L of 0.1 M hydrochloric acid (HC1) solution was added to 300 $\mu$L of the gold nanoparticle solution and the mixture was vortexed. 20 $\mu$L of the probe DNA solution after deprotection was added and the mixture was vortexed. After spinning down, the mixture was left to stand at room temperature for more than 15 hours, and the DNA was modified with gold nanoparticles. The prepared gold nanoparticle probes were stored at room temperature and out of direct sunlight. Then, DNA that was not immobilized was removed by centrifugation. After centrifugation to remove as much supernatant as possible, the gold nanoparticle probe was resuspended with the addition of a 0.1 M phosphate buffer (pH 7.0).

2. Preparation of Sample Mixture

The sample mixture was prepared by adding the probe solution created as described above to the extract obtained by applying a nucleic acid extraction treatment to the sample, further adding sodium chloride (NaCl) saturated aqueous solution and bovine serum albumin (BSA) thereto, and applying heat at 95°C thereto. The BSA concentrations were set to four types: 0 mg/L (no addition); 0.01 mg/L; 0.1 mg/L; and 1 mg/L, with respect to the mixture (reagent composition in assay systems) containing the sample extract, the probe solution, the sodium chloride saturated aqueous solution, and the BSA. For the samples, nucleic acid concentration was measured separately by the official method (PCR method).

3. Measurement and Evaluation of Absorbance

The absorption spectrum of the heated sample mixture was measured and the absorbance ratio $R_{650}/R_{550}$ was calculated by dividing the absorbance at the 650 nm wavelength by the absorbance at the 550 nm wavelength.

A strong correlation was observed between the absorbance ratio in the case of no BSA addition and the target nucleic acid concentration measured by the official method for the same sample. Accordingly, it was confirmed that the target nucleic acid concentration in the sample could be quantified by the detection method according to the first embodiment.

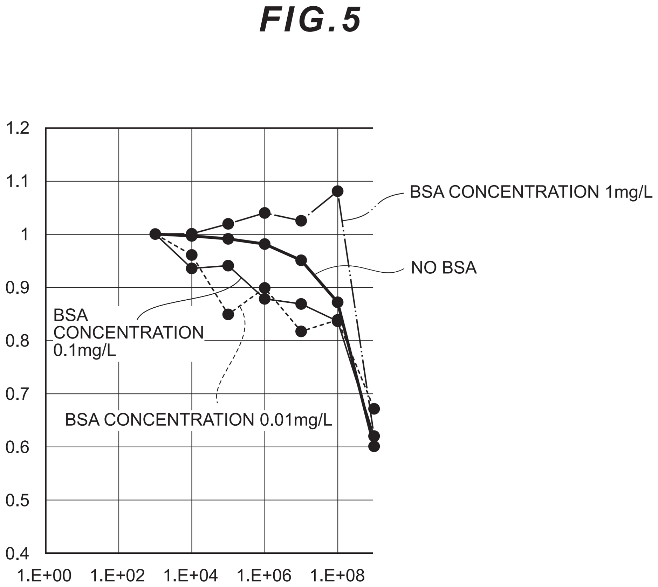

FIG. 5 is a graph showing the experimental results of Example 1. In FIG. 5, the horizontal axis shows the target nucleic acid concentration in the sample, and the vertical axis shows the sensor output (absorbance ratio $R_{650}/R_{550}$; the same applies hereinafter). As shown in FIG. 5, when BSA was not added, the measurable range of the target nucleic acid concentration was around $10^9$ to $10^6$. In contrast, when the BSA concentration was set to 0.1 mg/L, the measurable range was around $10^9$ to $10^3$, and thus, this allowed for the range to be extended.

On the other hand, when the BSA concentration was set to 0.01 mg/L, the sensor output was unstable. When the BSA concentration was set to 1 mg/L, the sensor output moved in the opposite direction to the nucleic acid concentration.

Based on the results described above, it was found that adding an appropriate amount of BSA allowed for the measurable range of target nucleic acid concentration to be extended. The appropriate amount (concentration) of BSA added in the sample mixture was found to be greater than 0.01 mg/L and less than 1 mg/L, and preferably in the vicinity of 0.1 mg/L.

(Second Embodiment)

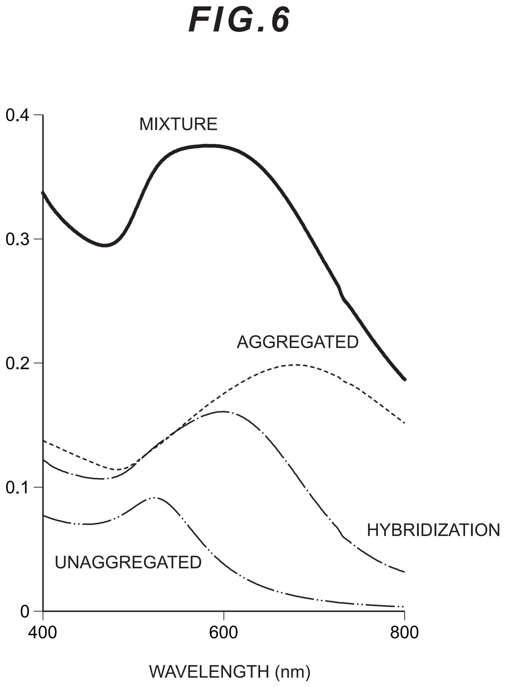

Next, a nucleic acid detection method according to a second embodiment of the present invention will be described. FIG. 6 is a conceptual diagram for describing a nucleic acid detection method in the second embodiment.

In the second embodiment, the curve fitting technique is used to estimate the nucleic acid concentration (see step 40 in FIG. 1). It should be noted that, in the second embodiment, addition of BSA as in the first embodiment is not essential when adding sodium chloride to the mixture of the sample extract and the probe solution (see step S20 in FIG. 1).

Here, gold nanoparticle probes may take three states: unaggregated, aggregated, and hybridized. In these states, gold nanoparticle probes each have a specific absorption spectrum. Therefore, if the target nucleic acid is present in the sample, the hybridization inhibits the aggregation of gold nanoparticles in the mixture of the sample extract and the probes (see the sample mixture, preparation step S20), resulting in changes in the absorption spectrum. Therefore, by performing spectral decomposition on the absorption spectrum of the sample mixture by means of curve fitting, the absorption spectrum specific to the hybridized gold nanoparticle probes can be calculated.

In detail, the absorption spectrum of the sample mixture can be expressed as a linear combination of the absorption spectra in the three states: unaggregated, aggregated, and hybridized, as shown in Equation (1):

$$X(\lambda) = x \times C(\lambda) + y \times D(\lambda) + z \times E(\lambda) \ \dots \ (1)$$

In Equation 1, $X(\lambda)$ is the absorbance of the sample mixture at wavelength $\lambda$, $D(\lambda)$ is the absorbance of the unaggregated probes at wavelength $\lambda$, $C(\lambda)$ is the absorbance of the aggregated probes at wavelength $\lambda$, and $E(\lambda)$ is the absorbance of the hybridized probes at wavelength $\lambda$. x, y, and z are coefficients.

According to the Lambert-Beer law, in Equation (1) above, the coefficients x, y, and z are considered to be proportional to the concentrations of gold nanoparticle probes in the unaggregated, aggregated, and hybridized states, respectively. Therefore, specifying these coefficients x, y, and z allows for the target nucleic acid concentration in the extract to be quantified. Specifically, the coefficients x, y, and z are determined so that the sum of the linear combinations shown in Equation (2) is minimal.

$$\sum_{l=400}^{800} [X(\lambda) - \{x \cdot C(\lambda) + y \cdot D(\lambda) + z \cdot E(\lambda)\}]^2$$

$$\cdots (2)$$

A practical technique includes measuring: (I) the absorption spectrum of the gold nanoparticle probe solution (hereinafter referred to as the "probe solution"), which does not contain any sample, NaCl, or BSA; (II) the absorption spectrum of the solution obtained by adding NaCl to the probe solution without samples and applying heat thereto (hereinafter referred to as the "blank solution"); and (III) the absorption spectrum of the solution obtained by mixing the nucleic acid extract from the sample and the probe solution, and adding NaCl and applying heat thereto (hereinafter referred to the "sample mixture").

If $A(\lambda)$ is the term representing the absorption spectrum (absorbance at wavelength $\lambda$) of the probe solution, $B(\lambda)$ is the term representing the absorption spectrum (ibid.) of the blank solution, and $S(\lambda)$ is the term representing the absorption spectrum (ibid.) of the sample mixture, then the linear combination $X'(\lambda)$ of these three absorption spectra is expressed by Equation (2):

$$X'(\lambda) = a \times A(\lambda) + b \times B(\lambda) + h \times S(\lambda) \ \dots \ (2)$$

The terms $A(\lambda)$, $B(\lambda)$, and h are determined using a least squares method so that this linear combination $X'(\lambda)$ is closest to the absorption spectrum $S(\lambda)$ of the sample mixture.

Here, as mentioned above, the absorption spectrum of the sample mixture can be said to be the linear combination of the absorption spectra of the gold nanoparticle probes in the unaggregated state, the aggregated state, and the hybridized state (see Equation (1)). The respective absorption spectra $A(\lambda)$, $B(\lambda)$, and $S(\lambda)$ of the probe solution, the blank solution, and the sample mixture can then be expressed using coefficients c, d, and e as in Equations (3) to (5).

$$A(\lambda) = C(\lambda) \ \dots \ (3)$$

$$B(\lambda) = c \times C(\lambda) + D(\lambda) \ \dots \ (4)$$

$$S(\lambda) = d \times C(\lambda) + e \times D(\lambda) + E(\lambda) \ \dots \ (5)$$

From Equations (3) to (5), the linear combination $X'(\lambda)$ can be expressed as Equation (6):

$$X'(\lambda) = (x-yc-dz-cez) \times A(\lambda) + (y-cz) \times B(\lambda) + z \times S(\lambda) \ \dots \ (6)$$

Therefore, the coefficient h in Equation (2) is equal to the coefficient z in Equation (1), and by determining the coefficient h, the hybridized gold nanoparticle probe concentration in the sample mixture, in other words, the target nucleic acid concentration, can be estimated.

(Example 2)

Gold nanoparticles with 5 nm diameter, OD 1, stabilized in suspension in a citrate buffer (Sigma-Aldrich) were used. The probe DNAs were T50-cCTO189F and T50-RT1R, including T50 spacers, based on the base sequences of the PCR primer (CTO189F A/B/C and RT1R) that detect the 16S rRNA gene of beta-proteobacterial ammonia oxidizing bacteria (AOB). Gold nanoparticle probes were prepared in which these probe DNAs were modified with gold nanoparticles.

A mixture totaling 51 μL was prepared by mixing 10 μL of T50-cCTO189F and 10 μL of T50-RT1R, which are gold nanoparticle probes, 1 μL of nucleic acid extract from the sample, and 30 μL of NaCl aqueous solution. This mixture was then heated at 95°C for 30 seconds and then at 59°C for 1 minute. Using this as the sample mixture, the absorption spectrum of the sample mixture was measured using an ultraviolet-visible spectrophotometer. For the samples, the absorption spectra were measured for each sample collected from multiple plants, and then the absorption spectrum of any one sample was selected and used for the subsequent calculations.

The absorption spectra of the probe solution and the blank solution were also measured in the same way. The blank solution was prepared and measured three times, and the detection limit was determined.

As mentioned above, the terms $A(\lambda)$, $B(\lambda)$, and h were determined using the least squares method so that the linear combination $X'(\lambda)$ of Equation (2) was closest to the absorption spectrum $S(\lambda)$ of the sample mixture. Using the coefficient h as an index for hybridization, a comparison was made with the target nucleic acid concentration of the sample measured by the qPCR method.

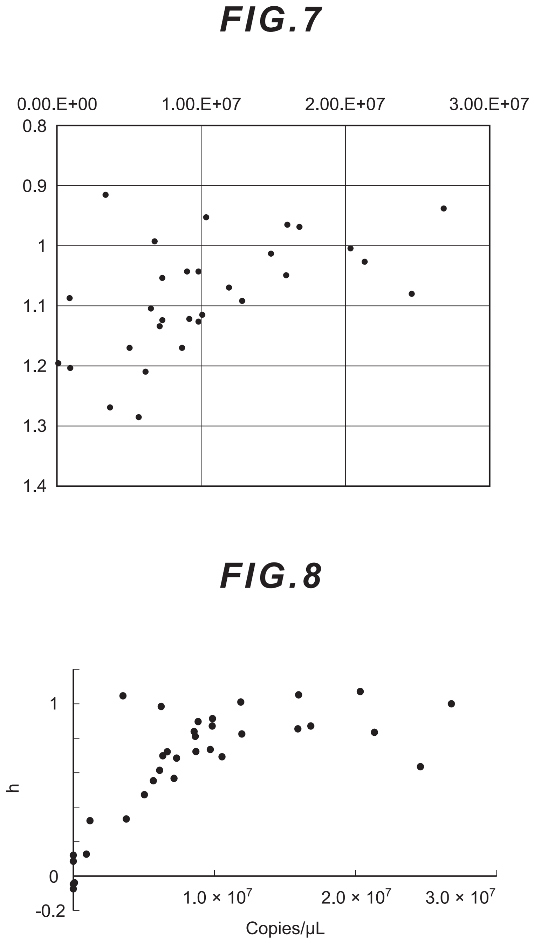

FIG. 7 is a graph showing a comparative example of Example 2, and it shows the relationship between the absorbance ratio calculated based on the absorption spectrum measured for the sample mixture and the target nucleic acid concentration measured by qPCR for the same sample mixture. In FIG. 7, the horizontal axis shows the target nucleic acid concentration (copies/$\mu$L), and the vertical axis shows the absorbance ratio. FIG. 8 is a graph showing the experimental results of Example 2, and it shows the relationship between the coefficients calculated by the curve fitting mentioned above and the target nucleic acid concentration measured by qPCR. In FIG. 8, the horizontal axis shows the target nucleic acid concentration, and the vertical axis shows the coefficient h.

As shown in FIG. 7, the correlation between the absorbance ratio and the target nucleic acid concentration weakened when the concentration fell below $1 \times 10^7$ (copies/$\mu$L). Therefore, it can be said that quantification using the method based on the absorbance ratio is difficult if the target nucleic acid concentration is low.

On the other hand, as shown in FIG. 8, in the case where the coefficient h was used, a positive correlation was observed between the coefficient h and the target nucleic acid concentration if the target nucleic acid concentration was lower than or equal to $1 \times 10^7$ (copies/$\mu$L). It should be noted that, if the target nucleic acid concentration was higher than or equal to this, the coefficient h shifted between 0.8 and 1.0.

For target nucleic acid concentrations lower than or equal to $1 \times 10^7$ (copies/$\mu$L), the coefficient h appears to be roughly proportional to the target nucleic acid concentration. In this region, by approximating the experimental results by a straight line, and the function shown in Equation (7) is obtained.

$$\text{H} = 9.85 \times 10^{-8} \times (\text{concentration}) + 0.010 \ \dots \ (7)$$

It should be noted that $\sigma = 0.976831613$. From this, it can be considered that the coefficient h is a number following the Lambert-Beer law.

For the blank solution (n = 3), the coefficients h were obtained by curve fitting and the mean was $5.9 \times 10^{-6}$ and the standard deviation ($\sigma$) was 0.086. From this, the detection limit ($3\sigma$) was calculated to be the coefficient h = 0.258 and $2.9 \times 10^6$ for the target concentration. From this, it can be said that the dynamic range in which the target nucleic acid concentration can be quantified is at least in the range from $2.9 \times 10^6$ to $10 \times 10^6$, inclusive.

Based on the results described above, it was found that the curve fitting technique of the absorption spectrum in the second embodiment could quantify the target nucleic acid concentration more accurately and to a lower concentration range than the conventional absorbance ratio.

In Example 2, BSA was not added to the sample mixture, but the target nucleic acid concentration may also be quantified using the curve fitting technique after adding BSA to the sample mixture. In such case, BSA will also be added to the blank solution. This will allow for the dynamic range to be extended even further to include the lower concentration side.

(Variation)

The reagent composition used in the first or second embodiment described above may also be used as a reagent kit for detecting microorganisms in the sample. The reagent kit comprises a probe solution and a sodium chloride solution. The probe solution is a gold nanoparticle probe solution in which DNA that binds specifically to a target nucleic acid is modified with gold nanoparticles. Bovine serum albumin may also be included in the reagent kit. The reagent kit may be used as a set enclosing each of these reagents in a predetermined amount. Such reagent kit can be used to prepare the sample mixture. The reagent kit can also be used to prepare the blank solution, and the probe solution included in the kit can also be used as is to measure the absorption spectrum. This allows for the estimation of the target nucleic acid concentration in the sample to be made by calculations based on the absorption spectra of the sample mixture, the blank solution, and the probe solution.

(Third Embodiment)

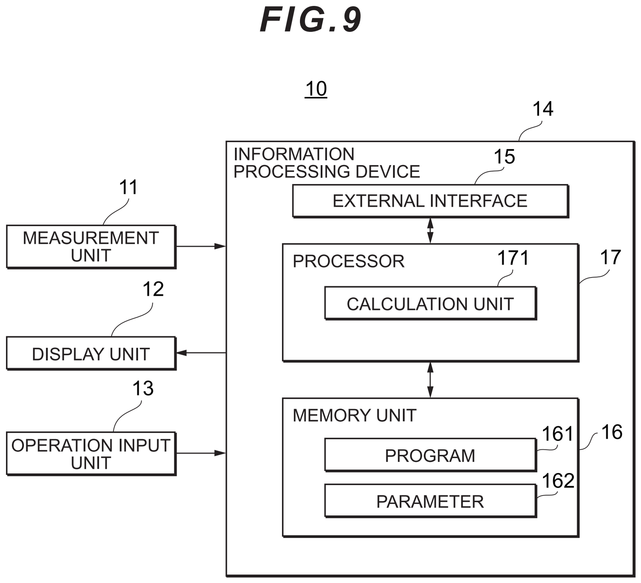

FIG. 9 is a block diagram illustrating a schematic configuration of a measurement system according to a third embodiment of the present invention. The measurement system 10 according to the third embodiment detects the microbial nucleic acid in a sample, and comprises a measurement unit 11, a display unit 12, an operation input unit 13, and an information processing device 14.

The measuring unit 11 is, for example, an absorptiometer, and it measures the absorption spectrum of the liquid containing the sample. The wavelength range of the absorption spectrum should be from 450 nm to 800 nm. Of course, the scattering light may be measured instead of the absorption spectrum.

Alternatively, a microplate reader may be used as the measuring unit 11. In this case, only two wavelengths around 550 nm and 650 nm may be set to be measured. Alternatively, a monochrome reader may be used to measure the desired multiple wavelengths.

The display unit 12 is, for example, a liquid crystal display or an organic EL display, and displays the absorption spectra input from the measurement unit 11, the target nucleic acid concentration in the sample estimated based on the absorption spectra, and the like.

The operation input unit 13 is an input device, such as a keyboard, mouse, or touch panel provided on the surface of the display unit 12, and it receives the operations by the user and inputs the signals corresponding to the operations to the below-mentioned processor 17.

The information processing device 14 is configured by general-purpose calculational processing procedures, such as a personal computer or tablet terminal, and it can be used as a measuring device to measure the target nucleic acid concentrations. As shown in FIG. 3, the information processing device 14 comprises an external interface 15, a memory unit 16, and a processor 17.

The external interface 15 connects the information processing device 14 to an external device, and it transmits and receives signals between the information processing device 14 and the external device. External devices connected to the information processing device 14 include the measurement unit 11, the display unit 12, the operation input unit 13, and other units. The external interface 11 functions, for example, as a receiving unit to receive the input of information representing the absorption spectra measured by the measuring unit 11.

The memory unit 16 is configured by using computer-readable memory media such as semiconductor memory, including ROM and/or RAM, and hard disks, and it stores, in addition to operating system programs and driver programs, application programs for executing various functions, various parameters to be used during the execution of such programs, and/or other kinds of data. For example, the memory unit 16 stores the program 161 that estimates the target nucleic acid concentration in the sample based on the absorption spectra represented by the information input from the measurement unit 11, and the parameters to be used in this estimation calculation. For example, the memory unit 16 may also store at least one of the absorption spectrum of the gold nanoparticle probe solution (probe solution) that does not contain any sample, NaCl, or BSA, or the absorption spectrum of the blank solution in which NaCl (or NaCl and BSA) is (are) added to the probe solution. In addition, the absorption spectra relating to various target nucleic acids may be stored as the absorption spectra of the probe solutions and/or the blank solutions, and the absorption spectra to be used may be read out depending on the bacteria and/or viruses to be detected.

The processor 17 is configured by using, for example, a central processing unit (CPU) and/or a graphics processing unit (GPU), and it performs an overall control of the respective units in the information processing device 14 by reading various programs stored in the memory unit 16, and it executes applications using the various programs. In particular, the calculation unit 171 of the processor 17 executes, when it receives the absorption spectrum (or intensities of multiple wavelengths) of the sample (i.e., the sample mixture) input from the measuring unit 11, the process of estimating the target nucleic acid concentration in the sample based on the absorption spectrum (or intensities of multiple wavelengths).

As one calculational processing, the calculation unit 171 can estimate the target nucleic acid concentration based on the absorbance ratio of the absorbances at 550 nm and 650 nm in the absorption spectrum of the sample mixture.

As another calculational processing, the calculation unit 171 can estimate the target nucleic acid concentration based on the absorption spectra of the probe solution, the blank solution, and the sample mixture. In particular, the calculation unit 171 can perform curve fitting based on the absorption spectra of the probe solution, the blank solution, and the sample mixture. In this case, the calculation unit 171 may acquire the absorption spectra of the probe solution, the blank solution, and the sample mixture measured by the measurement unit 11 from the measurement unit 11, and perform calculations based on these absorption spectra. Alternatively, the calculation unit 171 may acquire the absorption spectrum of the sample mixture from the measurement unit 11, read the absorption spectra of the probe solution and the blank solution from the memory unit 16, and perform calculations based on the absorption spectra of the sample mixture, the probe solution, and the blank solution. Of course, the calculation unit 171 may read the absorption spectrum of one of the probe solution or the blank solution from the memory unit 16, acquire the absorption spectrum of the other from the measurement unit 11, and perform calculations.

As explained above, according to each embodiment of the present invention, the nucleic acid concentration in the sample can be quantified. Samples are not particularly limited, and any analyte taken from sewage, wastewater, environmental waters, such as lakes, rivers and oceans, food, and people and animals, can be tested. In addition, the types of microorganisms are not particularly limited, and, for example, various microbial nucleic acids shown in Table 1 described above can be detected.

In addition, according to each embodiment of the present invention, nucleic acid concentration can be estimated quickly and easily, and even with the equivalent accuracy to the PCR method by using gold nanoparticle probes. In particular, in each embodiment of the present invention, since it is not necessary to apply any nucleic acid amplification treatment to the nucleic acid extract from the sample, the testing time can be significantly reduced. Specifically, the time required for the analytical reaction to the nucleic acid extract can be reduced to only 10 minutes, or to about 20 minutes even when considering the total time including the nucleic acid extraction, which is a pretreatment process.

Further, according to the first embodiment of the present invention, by adding BSA, the measurable dynamic range of the nucleic acid concentration can be extended to include the lower concentration side.

Moreover, according to the second embodiment of the present invention, by using the curve fitting technique, the estimation accuracy of the nucleic acid concentration can be improved, and the measurable dynamic range of the nucleic acid concentration can be extended to include the lower concentration side. In addition, by adding BSA and using the curve fitting technique, the dynamic range on the lower concentration side can be further extended.

According to each of these first and second embodiments, the nucleic acid concentration measurable dynamic range is allowed to be extended to include the lower concentration side to ensure a sufficient range. It is therefore possible to apply these embodiments by measuring the presence and/or concentration of microorganisms in environmental waters, and determining whether usage for drinking water and living water is possible, thereby, making it possible to improve accessibility to safe water in, for example, developing countries, disaster areas, and the like, can be improved.

In addition, according to the first to third embodiments described above, because gold nanoparticle probes are used; animal immunization is not required, thereby allowing manufacturing cost to be lowered compared to detection systems using antibodies; and chemical synthesis is possible, thereby allowing manufacturing to be stable. In addition, because expensive enzymes and analytical instruments such as in PCR are not required, tests can be conducted in cases where testing needs to be conducted in a regular or frequent manner, such as in microbial monitoring in environmental water, and/or in situations where facilities are not available, such as in developing countries or disaster areas.

According to the first to third embodiments described above, it is possible to detect various bacterial and viral genes as targets, in addition to the 16S rRNA gene of bacteria and the GI or GII type RNA gene of norovirus. In short, each embodiment of the present invention can be applied as long as gold nanoparticle probes can be prepared, in which a complementary DNA strand that binds specifically to a target nucleic acid is modified with gold nanoparticles.

The present invention as thus far described is not limited to the above-described first to third embodiments and variation, and various inventions can be formed by appropriately combining multiple components disclosed in the above-described first to third embodiments and variation. For example, such various inventions may be formed by excluding certain components from all of the components shown in the above-described first to third embodiments and variation, or by appropriately combining the components shown in the above-described first to third embodiments and variation.

(Appendix 1)

A microbial nucleic acid detection method, comprising the steps of: preparing a sample mixture by: mixing a probe solution, being a gold nanoparticle probe solution in which DNA that binds specifically to a target nucleic acid is modified with gold nanoparticles, with an extract obtained by applying a nucleic acid extraction treatment to a sample; and adding sodium chloride and bovine serum albumin and applying heat thereto;

acquiring an absorption spectrum of the sample mixture; and
performing calculation to estimate the concentration of the target nucleic acid in the sample based on the absorption spectrum.

(Appendix 2)

The microbial nucleic acid detection method according to Appendix 1, wherein the concentration of bovine serum albumin is greater than 0.01 mg/L and less than 1 mg/L in the sample mixture.

(Appendix 3)

The microbial nucleic acid detection method according to Appendix 1 or 2, wherein, in the calculation step, absorbances of the sample mixture at 550 nm and 650 nm are acquired and the concentration of the target nucleic acid is estimated based on an absorbance ratio of the absorbance at 650 nm to the absorbance at 550 nm.

DESCRIPTION OF REFERENCE NUMBERS

**[0110]** 1: Gold nanoparticle probe (probe), 2: Gold nanoparticle, 3: DNA, 10: Measurement system, 11: Measurement unit, 12: Display unit, 13: Operation input unit, 14: Information processing device, 15: External interface, 16: Memory unit, 17: Processor, 161: Program, 171: Calculation unit

**Claims**

1. A microbial nucleic acid detection method, comprising the steps of:

preparing a sample mixture by:

mixing a probe solution, being a gold nanoparticle probe solution in which DNA that binds specifically to a target nucleic acid is modified with gold nanoparticles, with an extract obtained by applying a nucleic acid extraction treatment to a sample; and

adding at least sodium chloride and applying heat thereto;

acquiring an absorption spectrum of the probe solution;

acquiring an absorption spectrum of a blank solution, being a sample-free solution obtained by adding sodium chloride to the probe solution and applying heat thereto;

acquiring an absorption spectrum of the sample mixture; and

performing calculations to estimate the concentration of the target nucleic acid in the sample based on the absorption spectra of the probe solution, the blank solution and the sample mixture.

2. The microbial nucleic acid detection method according to claim 1, wherein, in the calculation step, the concentration of the target nucleic acid in the sample is estimated by performing curve fitting based on the absorption spectra of the probe solution, the blank solution, and the sample mixture.

3. The microbial nucleic acid detection method according to claim 2, wherein, in the calculation step, a least squares method is performed so that a linear combination of a first term representing the absorption spectrum of the probe solution, a second term representing the absorption spectrum of the blank solution, and a third term representing the absorption spectrum of the sample mixture is closest to the third term representing the absorption spectrum of the sample mixture, and the concentration of the target nucleic acid is estimated based on a coefficient of the third term calculated by the least squares method.

4. The microbial nucleic acid detection method according to any one of claims 1 to 3, wherein the preparation step includes further adding bovine serum albumin.

5. The microbial nucleic acid detection method according to claim 4, wherein the concentration of the bovine serum albumin is greater than 0.01 mg/L and less than 1 mg/L in the sample mixture.

6. A reagent composition to be used for detecting a microorganism in a sample, comprising:

a probe solution, being a gold nanoparticle probe solution in which DNA that binds specifically to a target nucleic acid is modified with gold nanoparticles; and

a sodium chloride solution,

wherein

the reagent composition is used to prepare:

a sample mixture obtained by mixing the probe solution with an extract obtained by applying a nucleic acid extraction treatment to the sample, and adding at least sodium chloride and applying heat thereto; and

a blank solution, being a sample-free solution obtained by adding sodium chloride to the probe solution and applying heat thereto, and

the reagent composition is for estimating the concentration of the target nucleic acid in the sample by calculations based on absorption spectra of the sample mixture, the blank solution, and the probe solution.

7. The reagent composition according to claim 6, further comprising bovine serum albumin.

8. A reagent kit to be used for detecting a microorganism in a sample, comprising:

a probe solution, being a gold nanoparticle probe solution in which DNA that binds specifically to a target nucleic acid is modified with gold nanoparticles; and

a sodium chloride solution,

wherein

each of the probe solution and the sodium solution is enclosed in a predetermined amount, and

the reagent kit is used to prepare:

a sample mixture obtained by mixing the probe solution with an extract obtained by applying a nucleic acid extraction treatment to the sample, and adding at least sodium chloride and applying heat thereto; and

a blank solution, being a sample-free solution obtained by adding sodium chloride to the probe solution and applying heat thereto, and

the reagent kit is for estimating the concentration of the target nucleic acid in the sample by calculations based on absorption spectra of the sample mixture, the blank solution, and the probe solution.

**9.** The reagent kit according to claim 8, further comprising bovine serum albumin enclosed in a predetermined amount.

**10.** A measurement system, comprising:

a measurement unit that acquires an absorption spectrum of a sample mixture, the sample mixture being obtained by mixing a probe solution, being a gold nanoparticle probe solution in which DNA that binds specifically to a target nucleic acid is modified with gold nanoparticles, with an extract obtained by applying a nucleic acid extraction treatment to a sample, and adding at least sodium chloride and applying heat thereto; and
a calculation unit that estimates the concentration of the target nucleic acid in the sample based on an absorption spectrum of the probe solution, an absorption spectrum of a blank solution, being a sample-free solution obtained by adding sodium chloride to the probe solution and applying heat thereto, and the absorption spectrum of the sample mixture.

**11.** The measurement system according to claim 10, wherein

the measurement unit further measures an absorption spectrum of the probe solution and an absorption spectrum of the blank solution, and
the calculation unit estimates the concentration of the target nucleic acid in the sample based on the absorption spectrum of the probe solution, the absorption spectrum of the blank solution, and the absorption spectrum of the sample mixture, all of which are measured by the measurement unit.

**12.** The measurement system according to claim 10, further comprising

a memory unit that stores at least one of the absorption spectrum of the probe solution or the absorption spectrum of the blank solution, and wherein
the calculation unit reads at least one of the absorption spectrum of the probe solution or the absorption spectrum of the blank solution from the memory unit, and estimates the concentration of the target nucleic acid in the sample using the read absorption spectrum.

**13.** The measurement system according to any of claims 10 to 12, wherein the calculation unit estimates the concentration of the target nucleic acid in the sample by performing curve fitting based on the absorption spectra of the probe solution, the blank solution and the sample mixture.

**14.** A program for causing a computer to perform:

a step of acquiring an absorption spectrum of a sample mixture, the sample mixture being obtained by mixing a probe solution, being a gold nanoparticle probe solution in which DNA that binds specifically to a target nucleic acid is modified with gold nanoparticles, with an extract obtained by applying a nucleic acid extraction treatment to a sample, and adding at least sodium chloride and applying heat thereto; and
a step of estimating the concentration of the target nucleic acid in the sample based on an absorption spectrum of the probe solution, an absorption spectrum of a blank solution, being a sample-free solution obtained by adding sodium chloride to the probe solution and applying heat thereto, and the absorption step of the sample mixture.

# FIG.1

```
          ┌─────────────┐
          │    START    │
          └──────┬──────┘
                 │
                 ▼
   ┌─────────────────────────┐
   │  NUCLEIC ACID EXTRACTION │ ── S10
   │        TREATMENT         │
   └────────────┬────────────┘
                │
                ▼
   ┌─────────────────────────┐
   │  ADD DNA PROBE SOLUTION  │
   │  TO EXTRACT, FURTHER ADD │ ── S20
   │    NaCl AND APPLY HEAT   │
   └────────────┬────────────┘
                │
                ▼
   ┌─────────────────────────┐
   │   ACQUIRE ABSORPTION     │ ── S30
   │        SPECTRUM          │
   └────────────┬────────────┘
                │
                ▼
   ┌─────────────────────────┐
   │  ESTIMATE NUCLEIC ACID   │ ── S40
   │      CONCENTRATION       │
   └────────────┬────────────┘
                │
                ▼
          ┌─────────────┐
          │     END     │
          └─────────────┘
```

## FIG.2

# FIG.3

SAMPLE AFTER
NUCLEIC ACID
EXTRACTION

TARGET

HIGH
CONCENTRATION

NaCl

NO TARGET

# FIG.4

# FIG.5

# FIG.6

# FIG.7

# FIG.8

# FIG.9

10

INFORMATION PROCESSING DEVICE 14

15 EXTERNAL INTERFACE

11 MEASUREMENT UNIT

PROCESSOR 17
171 CALCULATION UNIT

12 DISPLAY UNIT

13 OPERATION INPUT UNIT

MEMORY UNIT 16
161 PROGRAM
162 PARAMETER

## INTERNATIONAL SEARCH REPORT

| International application No. |
| --- |
| **PCT/JP2024/015385** |

**A. CLASSIFICATION OF SUBJECT MATTER**

*C12Q 1/6888*(2018.01)i; *C12M 1/34*(2006.01)i
FI: C12Q1/6888 Z; C12M1/34 B

According to International Patent Classification (IPC) or to both national classification and IPC

**B. FIELDS SEARCHED**

Minimum documentation searched (classification system followed by classification symbols)

C12Q1/6888; C12M1/34

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| A | US 2020/0132693 A1 (BOARD OF TRUSTEES OF MICHIGAN STATE UNIVERSITY) 30 April 2020 (2020-04-30)<br>claims, examples, fig. 1 | 1-14 |
| A | JP 2007-071667 A (HOKKAIDO UNIV) 22 March 2007 (2007-03-22)<br>claims, examples | 1-14 |
| A | NAKAJIMA, M. et al. Simple assay for colorimetric quantification of unamplified bacterial 16S rRNA in activated sludge using gold nanoprobes. Chemosphere. 2021, vol. 263, article no. 128331<br>abstract, Scheme s1, 2.5. | 1-14 |

☐ Further documents are listed in the continuation of Box C.    ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **21 June 2024** | **02 July 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
### Information on patent family members

International application No.

**PCT/JP2024/015385**

| Patent document cited in search report | Publication date (day/month/year) | Patent family member(s) | Publication date (day/month/year) |
|---|---|---|---|
| US 2020/0132693 A1 | 30 April 2020 | WO 2018/170348 A1 claims, examples, fig. 1 | |
| JP 2007-071667 A | 22 March 2007 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2021122186 A

- JP 2021158989 A

### Non-patent literature cited in the description

- **MERI NAKAJIMA et al.** Development of a Paper-based Analytical Chip for the Detection of Bacterial 16S rRNA in Wastewater Samples. *BUNSEKI KAGAKU*, 2020, vol. 69 (12), 715-722
- **MERI NAKAJIMA et al.** Simple Assay for Colorimetric Quantification of Unamplified Bacterial 16S rRNA in Activated Sludge using Gold Nanoprobes. *Chemosphere*, January 2021, vol. 263, 128331
- **HISASHI SATOH et al.** Highly sensitive and homogeneous detection of unamplified RNA based on the light scattering properties of gold nanoparticle probes. *Biosensors and Bioelectronics: X*, December 2022, vol. 12, 100249

- **LIU et al.** Preparation of aptamer-linked gold nanoparticle purple aggregates for colorimetric sensing of analytes. *Nat. Protoc.*, vol. 1, 246e252, https://doi.org/10.1038/nprot.2006.38
- **X. ZHANG et al.** Instantaneous and Quantitative Functionalization of Gold Nanoparticles with Thiolated DNA Using a pH-Assisted and Surfactant-Free Route. *J. Am. Chem. Soc.*, 2012, vol. 134, 7266, https://doi.org/10.1021/ja3014055